# EUROPEAN PATENT APPLICATION

(11) **EP 4 215 622 A1**
(43) Date of publication of application: **26.07.2023**
(21) Application number: 21869428.9
(22) Date of filing: 16.09.2021
(51) Int. Cl.: C12Q 1/6876, C12N 15/09, C12Q 1/6851, G01N 33/50, G01N 33/53

(54) **METHOD FOR DETECTING INFANTILE ATOPIC DERMATITIS**

(30) Priority: 16.09.2020 JP 2020155444
(71) Applicant: Kao Corporation, Chuo-ku Tokyo 103-8210 (JP)
(72) Inventor: SHIMA, Kyoko, Haga-gun, Tochigi 321-3497 (JP); UEHARA, Yuya, Haga-gun, Tochigi 321-3497 (JP); INOUE, Takayoshi, Haga-gun, Tochigi 321-3497 (JP); TAKADA, Naoto, Haga-gun, Tochigi 321-3497 (JP)
(74) Representative: Vossius & Partner Patentanwälte Rechtsanwälte mbB
(86) International application number: PCT/JP2021/034174
(87) International publication number: WO 2022/059745

(57) **Abstract**

Provided is a method for detecting childhood atopic dermatitis using skin surface lipids. The method for detecting childhood atopic dermatitis in a test subject comprises a step of measuring an expression level of TARC gene or an expression product thereof in skin surface lipids collected from the test subject.

## Description

### Field of the Invention

The present invention relates to a method for detecting childhood atopic dermatitis using a marker for childhood atopic dermatitis derived from skin surface lipids.

### Background of the Invention

Atopic dermatitis (hereinafter, also referred to as "AD") is an eczematous skin disease which develops mainly in a person having an atopic predisposition. Typical symptoms of atopic dermatitis are chronic and recurrent itchiness, eruption, erythema, and the like which occur bilaterally and symmetrically, as well as incomplete keratinization, decline in barrier function, dry skin, and the like. Most cases of atopic dermatitis occur in childhood, and children tend to outgrow atopic dermatitis. However, the number of adult or intractable atopic dermatitis cases has also increased in recent years.

Newborns/infants having a genetic predisposition to have allergy or atopy are known to develop various allergic diseases such as infantile eczema, atopic dermatitis, food allergy, bronchial asthma, and allergic rhinitis with age (allergy march). For such allergic diseases, the development of one disease is likely to trigger another allergic disease, and the treatment thereof is often prolonged. Hence, the development of an allergic disease reportedly needs to be suppressed at the stage of childhood.

As methods for detecting atopic dermatitis using biomarkers, for example, the detection of peripheral blood eosinophil counts, total serum IgE values, LDH (lactate dehydrogenase) values, serum thymus and activation-regulated chemokine (TARC) values, or squamous cell carcinoma antigen 2 (SCCA2) has been proposed (Non Patent Literatures 1, 2 and 3). Among these biomarkers, the serum TARC has been reported to also reflect the exacerbation of pediatric atopic dermatitis (Non Patent Literature 4). The report states that in the determination of AD using the serum TARC as an index, the sensitivity and specificity of determination are reduced in pediatric subjects under the age of 2 compared with pediatric subjects at age 2 or older.

Meanwhile, techniques of examining the current and future *in vivo* physiological states of humans by the analysis of nucleic acids such as DNA or RNA in biological samples have been developed. Nucleic acids of biological origin can be extracted from body fluids such as blood, secretions, tissues, and the like, and it has recently been reported that: RNA contained in skin surface lipids (SSL) can be used as a biological sample for analysis; and marker genes of the epidermis, the sweat gland, the hair follicle and the sebaceous gland can be detected from SSL (Patent Literature 1). Few specific findings have been gained about the relation of marker gene expression in the SSL to marker gene expression in body fluids such as blood. It is uncertain whether a disease marker in serum is also capable of being expressed equivalently in SSL and serving as a marker with equivalent accuracy.
[Patent Literature 1] WO 2018/008319
[Non Patent Literature 1] Sugawara et al., Allergy (2002) 57: 180-181
[Non Patent Literature 2] Ohta et al., Ann Clin Biochem.(2012) 49: 277-84
[Non Patent Literature 3] Kato et al., The Japanese Journal of Dermatology (2018) 128: 2431-2502
[Non Patent Literature 4] Fujisawa et al., The Japanese Journal of Pediatric Allergy and Clinical Immunology (2005) 19(5): 744-752

### Summary of the Invention

The present invention relates to the following 1) to 3) .
1) A method for detecting childhood atopic dermatitis in a test subject, comprising a step of measuring an expression level of TARC gene or an expression product thereof in skin surface lipids collected from the test subject.
2) Use of TARC gene or an expression product thereof derived from skin surface lipids collected from a test subject as a marker for childhood atopic dermatitis.
3) A test kit for detecting childhood atopic dermatitis, the kit being used in a method according to 1), and comprising an oligonucleotide which specifically hybridizes to the gene, or an antibody which recognizes an expression product of the gene.

### Brief Description of Drawings

[Figure 1] Figure 1 shows the RNA expression level of TARC in SSL of healthy children (HL) and children with childhood atopic dermatitis (AD).
[Figure 2] Figure 2 shows the RNA expression level of TARC in SSL of healthy adult subjects (HL) and adult atopic dermatitis patients (AD).
[Figure 3] Figure 3 shows the protein level of TARC in the serum of healthy adult subjects (HL) and adult atopic dermatitis patients (AD).

### Detailed Description of the Invention

All patent literatures, non patent literatures, and other publications cited herein are incorporated herein by reference in their entirety.

The present invention relates to a provision of a method for detecting childhood atopic dermatitis using skin surface lipids.

The present inventors found that: the mRNA expression level of TARC contained in SSL collected from children having atopic dermatitis and children with healthy skin having no allergic predisposition significantly differ therebetween; and childhood atopic dermatitis can be detected on the basis of this index, and this index is particularly useful in the detection of low-grade and intermediate-grade atopic dermatitis.

The present invention enables childhood atopic dermatitis to be conveniently and noninvasively detected early with high accuracy, sensitivity and specificity.

In the present invention, the term "nucleic acid" or "polynucleotide" means DNA or RNA. The DNA includes all of cDNA, genomic DNA, and synthetic DNA. The "RNA" includes all of total RNA, mRNA, rRNA, tRNA, non-coding RNA and synthetic RNA.

In the present invention, the "gene" encompasses double-stranded DNA including human genomic DNA as well as single-stranded DNA (positive strand) including cDNA, single-stranded DNA having a sequence complementary to the positive strand (complementary strand), and their fragments, and means matter containing some biological information in sequence information on bases constituting DNA.

The "gene" encompasses not only a "gene" represented by a particular nucleotide sequence but a nucleic acid encoding a congener (i.e., a homolog or an ortholog), a variant such as gene polymorphism, and a derivative thereof.

In the present invention, the "TARC" refers to thymus and activation-regulated chemokine which is a chemokine having a leukocyte chemotactic activity and is a chemokine produced in epidermal keratinocytes, dendritic cells, lymphocytes, vascular endothelial cells, and the like, and is also called chemokin ligand 17 (CCL17). The TARC gene (mRNA) is registered under reference sequence No. "NM_002987.3" in NCBI.

In the present invention, the "expression product" of a gene conceptually encompasses a transcription product and a translation product of the gene. The "transcription product" is RNA resulting from the transcription of the gene (DNA), and the "translation product" means a protein which is encoded by the gene and translationally synthesized on the basis of the RNA.

In the present invention, the "atopic dermatitis" refers to a disease which has eczema with itch in principal etiology and repeats exacerbation and remission, and most of its patients reportedly have an atopic predisposition. Examples of the atopic predisposition include i) family history and/or previous medical history (any or a plurality of diseases among bronchial asthma, allergic rhinitis/ conjunctivitis, and atopic dermatitis), or ii) a predisposition to easily produce an IgE antibody.

In the present invention, the "child" conceptually includes a "pediatric" individual before the start of secondary sex characteristics, specifically a 12-year-old or younger pediatric individual, in the broad sense, and preferably refers to a child from the age of 0 to below school age, specifically, a 0- to 5-year-old child. Eruption of atopic dermatitis which develops in children is characterized by starting on the head or the face in infancy, often spreading down to the body trunk or the extremities, decreasing on the face in early childhood, and appearing mostly on the neck and joints of the extremities. In recent years, childhood atopic dermatitis and adult atopic dermatitis have been reported to differ in that abnormal epidermal keratinization associated with chronic inflammatory abnormality is observed in adult atopic dermatitis, compared with childhood atopic dermatitis (Journal of allergy and clinical immunology, volume 141, issue 6, June 2018, pageg 2094-2106), though it is uncertain due to a small number of reported cases.

The degree of progression (severity) of atopic dermatitis is classified into, for example, no symptoms, minor, mild (low grade), moderate (intermediate grade), and severe (high grade) depending on the state of eruption. For example, Eczema Area and Severity Index ("EASI" <Exp Dermatol, 2001; 10: 11-18>) and Investigator's Global Assessment ("IGA" <J Am Acad Dermatol, 2016; 74: 288-94>) are well known as severity classification.

For example, EASI is a score from 0 to 72 which is calculated on the basis of scores based on four symptoms, erythema, edema/oozing/papule, excoriation, and lichenification, in each of the head and neck, the body trunk, the upper limbs, and the lower limbs as assessed sites, and the percentage (%) of areas with the four symptoms based on the whole assessed sites. In IGA, a physician or a researcher serving as an evaluator comprehensively assesses the state of eruption for systemic or particular assessed sites and makes assessment on five scales "no symptom", "minor", "low grade", "intermediate grade" and "high grade". Six scales including "highest grade" may be used. The IGA scores can be used by conversion to ordinal scales such as "no symptom = 0", "minor = 1", "low grade = 2", "intermediate grade = 3", "high grade = 4" and "highest grade = 5".

In the present invention, the "detection of childhood atopic dermatitis" encompasses to elucidate the presence (with symptoms) or absence (without symptoms) of childhood atopic dermatitis as well as to elucidate the degree of progression, i.e., "low grade", "intermediate grade" and "high grade", of childhood atopic dermatitis. Preferred examples thereof include the detection of each of "without symptoms", "low grade", and "intermediate grade".

In this context, the "low grade" refers to a pathological condition composed mainly of dryness and low-grade erythema, scale, or the like and corresponds to an EASI score of larger than 0 and smaller than 6 or an IGA score of 2. The "intermediate grade" refers to a pathological condition composed mainly of erythema up to the intermediate grade, scale, a small number of papules, excoriation, or the like and corresponds to an EASI score of 6 or larger and smaller than 23 or an IGA score of 3. The "high grade" refers to a pathological condition composed mainly of erythema accompanied by high-grade puffiness/edema/oozing or lichenification, a rash of papule, high-grade scale, scabbing, vesicle, erosion, a large number of excoriation, prurigo nodule, or the like and corresponds to an EASI score of 23 or larger and 72 or smaller or an IGA score of 4. The "no symptom" refers to the same level as that in healthy children without symptoms as a result of remission or almost remission.

In the assessment of "no symptom", "low grade", "intermediate grade" and "high grade" using an EASI score or an IGA score, the range of the score based on which each assessment is made is not limited to those described above and can be appropriately determined.

In the present invention, the term "detection" may be used interchangeably with the term "examination", "measurement", "determination", "evaluation" or "assistance of evaluation". In the present specification, the term "detection", "examination", "determination" or "evaluation" does not include determination, evaluation or diagnosis by a physician.

As shown in Examples mentioned later, expression analysis of RNA in SSL was conducted for 20 healthy children and a total of 16 children with atopic dermatitis including 9 children diagnosed with mild (score: larger than 0 and smaller than 6) and 7 children diagnosed with moderate (score: 6 or larger and smaller than 23) (from 6 months after birth to 5 years old) in accordance with the severity of EASI. As a result, the expression of TARC gene was increased in a severity-dependent manner.

The discrimination precision between healthy children and children with mild to moderate atopic dermatitis, between healthy children and children with mild atopic dermatitis, and between children with mild atopic dermatitis and children with moderate atopic dermatitis was verified on the basis of RNA expression level data on TARC derived from SSL of healthy children, children with mild atopic dermatitis and children with moderate atopic dermatitis. As a result, accuracy ((true positive + true negative) / all samples) was 89%, 79% and 87%, respectively, indicating that discrimination is achieved with high precision.

According to the method of the present invention, even in the case of targeting children under the age of 2 for which discrimination by conventional methods has been considered difficult, the discrimination precision between healthy children and children with mild to moderate atopic dermatitis, between healthy children and children with mild atopic dermatitis, and between children with mild atopic dermatitis and children with moderate atopic dermatitis is 75%, 70% and 90%, respectively, in terms of accuracy, and favorable discrimination is thus achieved in all the cases.

Thus, the TARC gene or the expression product thereof in SSL is useful as a marker for childhood atopic dermatitis for detecting childhood atopic dermatitis and is particularly useful as a marker which distinguishably detects each of "no symptom of childhood atopic dermatitis", "low-grade or intermediate-grade childhood atopic dermatitis", "low-grade childhood atopic dermatitis", and "intermediate-grade childhood atopic dermatitis".

In the present invention, the TARC gene capable of serving as a marker for detecting childhood atopic dermatitis (hereinafter, also referred to as a "target gene") also encompasses a gene having a nucleotide sequence substantially identical to the nucleotide sequence of DNA constituting the TARC gene as long as the gene is capable of serving as a biomarker for detecting childhood atopic dermatitis. In this context, the substantially identical nucleotide sequence means a nucleotide sequence having 90% or higher, preferably 95% or higher, more preferably 98% or higher, further more preferably 99% or higher identity to the nucleotide sequence of DNA constituting the gene, for example, when searched using homology calculation algorithm NCBI BLAST under conditions of expectation value = 10; gap accepted; filtering = ON; match score = 1; and mismatch score = -3.

The method for detecting childhood atopic dermatitis according to the present invention includes a step of measuring an expression level of TARC gene or an expression product thereof for SSL collected from a test subject.

In the present invention, the test subject from which SSL are collected is not particularly limited by sex, race, and the like, as long as the test subject is a child. The test subject is preferably a child in need of the detection of atopic dermatitis, or a child suspected of developing atopic dermatitis. Examples of the test subject include children having atopic dermatitis, children having eczema with itch, children confirmed to have an atopic predisposition from a family history and/or a previous disease, and children having a predisposition to easily produce an IgE antibody.

In this context, the "skin surface lipids (SSL)" refer to a lipid-soluble fraction present on skin surface, and is also called sebum. In general, SSL mainly contain secretions secreted from the exocrine gland such as the sebaceous gland in the skin, and are present on skin surface in the form of a thin layer that covers the skin surface. SSL contain RNA expressed in skin cells (see Patent Literature 1 described above). In the present specification, the "skin" is a generic name for regions containing tissues such as the stratum corneum, the epidermis, the dermis, and the hair follicle as well as the sweat gland, the sebaceous gland and other glands, unless otherwise specified.

Any approach for use in the collection or removal of SSL from the skin can be adopted for the collection of SSL from the skin of a test subject. Preferably, an SSL-absorbent material or an SSL-adhesive material mentioned later, or a tool for scraping SSL from the skin can be used. The SSL-absorbent material or the SSL-adhesive material is not particularly limited as long as the material has affinity for SSL. Examples thereof include polypropylene and pulp. More detailed examples of the procedure of collecting SSL from the skin include a method of allowing SSL to be absorbed to a sheet-like material such as an oil blotting paper or an oil blotting film, a method of allowing SSL to adhere to a glass plate, a tape, or the like, and a method of collecting SSL by scraping with a spatula, a scraper, or the like. In order to improve the adsorbability of SSL, an SSL-absorbent material impregnated in advance with a solvent having high lipid solubility may be used. On the other hand, the SSL-absorbent material preferably has a low content of a solvent having high water solubility or water because the adsorption of SSL to a material containing the solvent having high water solubility or water is inhibited. The SSL-absorbent material is preferably used in a dry state. Examples of the site of the skin from which SSL are collected include, but are not particularly limited to, the skin at an arbitrary site of the body, such as the head, the face, the neck, the body trunk, and the limbs. A site having high secretion of sebum, for example, the facial skin, is preferred.

The RNA-containing SSL collected from the test subject may be preserved for a given period. The collected SSL is preferably preserved under low-temperature conditions as rapidly as possible after collection in order to minimize the degradation of contained RNA. The temperature conditions for the preservation of RNA-containing SSL according to the present invention can be 0°C or lower and are preferably from -20 ± 20°C to -80 ± 20°C, more preferably from -20 ± 10°C to -80 ± 10°C, further more preferably from -20 ± 20°C to -40 ± 20°C, further more preferably from -20 ± 10°C to -40 ± 10°C, further more preferably -20 ± 10°C, further more preferably -20 ± 5°C. The period of preservation of the RNA-containing SSL under the low-temperature conditions is not particularly limited and is preferably 12 months or shorter, for example, 6 hours or longer and 12 months or shorter, more preferably 6 months or shorter, for example, 1 day or longer and 6 months or shorter, further more preferably 3 months or shorter, for example, 3 days or longer and 3 months or shorter.

In the present invention, examples of the measurement object for the expression level of TARC gene which is a target gene or an expression product thereof include RNA, DNA encoding the RNA, a protein encoded by the RNA, a molecule which interacts with the protein, a molecule which interacts with the RNA, and a molecule which interacts with the DNA. RNA is preferred, and mRNA is more preferred. In this context, examples of the molecule which interacts with the RNA, the DNA or the protein include DNA, RNA, proteins, polysaccharides, oligosaccharides, monosaccharides, lipids, fatty acids, and their phosphorylation products, alkylation products, and sugar adducts, and complexes of any of them. The expression level comprehensively means the expression level (expressed amount) or activity of the gene or the expression product.

In a preferred aspect, in the method of the present invention, the expression level of mRNA contained in SSL is analyzed. Preferably, RNA is converted to cDNA through reverse transcription, followed by the measurement of the cDNA or an amplification product thereof.

In the extraction of RNA from SSL, a method which is usually used in RNA extraction or purification from a biological sample, for example, phenol/chloroform method, AGPC (acid guanidinium thiocyanate-phenol-chloroform extraction) method, a method using a column such as TRIzol(R), RNeasy(R), or QIAzol(R), a method using special magnetic particles coated with silica, a method using magnetic particles for solid phase reversible immobilization, or extraction with a commercially available RNA extraction reagent such as ISOGEN can be used.

In the reverse transcription, primers which target particular RNA to be analyzed may be used, and random primers are preferably used for more comprehensive nucleic acid preservation and analysis. In the reverse transcription, common reverse transcriptase or reverse transcription reagent kit can be used. Highly accurate and efficient reverse transcriptase or reverse transcription reagent kit is suitably used. Examples thereof include M-MLV reverse transcriptase and its modified forms, and commercially available reverse transcriptase or reverse transcription reagent kits, for example, PrimeScript(R) Reverse Transcriptase series (Takara Bio Inc.) and SuperScript(R) Reverse Transcriptase series (Thermo Fisher Scientific, Inc.). SuperScript(R) III Reverse Transcriptase, SuperScript(R) VILO cDNA Synthesis kit (both from Thermo Fisher Scientific, Inc.), and the like are preferably used.

The temperature of extension reaction in the reverse transcription is adjusted to preferably 42°C ± 1°C, more preferably 42°C ± 0.5°C, further more preferably 42°C ± 0.25°C, while its reaction time is adjusted to preferably 60 minutes or longer, more preferably from 80 to 120 minutes.

In the case of using RNA, cDNA or DNA as a measurement object, the method for measuring the expression level can be selected from nucleic acid amplification methods typified by PCR using DNA primers which hybridize thereto, real-time RT-PCR, multiplex PCR, SmartAmp, and LAMP, hybridization using a nucleic acid probe which hybridizes thereto (DNA chip, DNA microarray, dot blot hybridization, slot blot hybridization, Northern blot hybridization, and the like), a method of determining a nucleotide sequence (sequencing), and combined methods thereof.

In PCR, only TARC DNA to be analyzed may be amplified using a primer pair which targets TARC DNA, or a plurality of DNAs including TARC DNA may be amplified at the same time using a plurality of primer pairs. Examples of the method for amplifying only the DNA to be analyzed include RT-PCR. Examples of the method for amplifying a plurality of DNAs at the same time include multiplex PCR. The multiplex PCR can be carried out using a commercially available kit (e.g., Ion AmpliSeq Transcriptome Human Gene Expression Kit; Life Technologies Japan Ltd.).

The temperature of annealing and extension reaction in the PCR depends on the primers used and therefore cannot be generalized. In the case of using the multiplex PCR kit described above, the temperature is preferably 62°C ± 1°C, more preferably 62°C ± 0.5°C, further more preferably 62°C ± 0.25°C. Thus, preferably, the annealing and the extension reaction are performed by one step in the PCR. The time of the step of the annealing and the extension reaction can be adjusted depending on the size of DNA to be amplified, and the like, and is preferably from 14 to 18 minutes. Conditions for denaturation reaction in the PCR can be adjusted depending on the size of DNA to be amplified, and are preferably from 95 to 99°C and from 10 to 60 seconds. The reverse transcription and the PCR using the temperatures and the times as described above can be carried out using a thermal cycler which is generally used for PCR.

The reaction product obtained by the PCR is preferably purified by the size separation of the reaction product. By the size separation, the PCR reaction product of interest can be separated from the primers and other impurities contained in the PCR reaction solution. The size separation of DNA can be performed using, for example, a size separation column, a size separation chip, or magnetic beads which can be used in size separation. Preferred examples of the magnetic beads which can be used in size separation include magnetic beads for solid phase reversible immobilization (SPRI) such as Ampure XP.

The purified PCR reaction product may be subjected to further treatment necessary for conducting subsequent quantitative analysis. For example, for DNA sequencing, the purified PCR reaction product may be prepared into an appropriate buffer solution, the PCR primer regions contained in DNA amplified by PCR may be cleaved, and an adaptor sequence may be further added to the amplified DNA. For example, the purified PCR reaction product can be prepared into a buffer solution, and the removal of the PCR primer sequences and adaptor ligation can be performed for the amplified DNA. If necessary, the obtained reaction product can be amplified to prepare a library for quantitative analysis. These operations can be performed, for example, using 5 × VILO RT Reaction Mix attached to SuperScript(R) VILO cDNA Synthesis kit (Life Technologies Japan Ltd.), 5 × Ion AmpliSeq HiFi Mix attached to Ion AmpliSeq Transcriptome Human Gene Expression Kit (Life Technologies Japan Ltd.), and Ion AmpliSeq Transcriptome Human Gene Expression Core Panel according to a protocol attached to each kit.

In the case of measuring the expression level of target TARC gene or a nucleic acid derived therefrom by use of Northern blot hybridization, for example, probe DNA is first labeled with a radioisotope, a fluorescent material, or the like. Subsequently, the obtained labeled DNA is allowed to hybridize to biological sample-derived RNA transferred to a nylon membrane or the like in accordance with a routine method. Then, the formed duplex of the labeled DNA and the RNA is measured by detecting a signal derived from the label.

In the case of measuring the expression level of target TARC gene or a nucleic acid derived therefrom by use of RT-PCR, for example, cDNA is first prepared from biological sample-derived RNA in accordance with a routine method. This cDNA is used as a template, and a pair of primers (a positive strand which binds to the cDNA (- strand) and an opposite strand which binds to a + strand) prepared so as to be able to amplify target TARC gene of the present invention is allowed to hybridize thereto. Then, PCR is performed in accordance with a routine method, and the obtained amplified double-stranded DNA is detected. In the detection of the amplified double-stranded DNA, for example, a method of detecting labeled double-stranded DNA produced by the PCR using primers labeled in advance with RI, a fluorescent material, or the like can be used.

In the case of measuring the expression level of target TARC gene or a nucleic acid derived therefrom by use of a DNA microarray, for example, an array in which at least one nucleic acid (cDNA or DNA) derived from the target gene of the present invention is immobilized on a support is used. Labeled cDNA or cRNA prepared from mRNA is allowed to bind onto the microarray, and the expression level of the mRNA can be measured by detecting the label on the microarray.

The nucleic acid to be immobilized in the array can be a nucleic acid which specifically hybridizes (i.e., substantially only to the nucleic acid of interest) under stringent conditions, and may be, for example, a nucleic acid having the whole sequence of the target gene of the present invention or may be a nucleic acid consisting of a partial sequence thereof. In this context, examples of the "partial sequence" include nucleic acids consisting of at least 15 to 25 bases. In this context, examples of the stringent conditions can usually include washing conditions on the order of "1 × SSC, 0.1% SDS, and 37°C". Examples of the more stringent hybridization conditions can include conditions on the order of "0.5 × SSC, 0.1% SDS, and 42°C". Examples of the much more stringent hybridization conditions can include conditions on the order of "0.1 × SSC, 0.1% SDS, and 65°C". The hybridization conditions are described in, for example, J. Sambrook et al., Molecular Cloning: A Laboratory Manual, Thrd Edition, Cold Spring Harbor Laboratory Press (2001).

In the case of measuring the expression level of the target gene or a nucleic acid derived therefrom by sequencing, examples thereof include analysis using a next-generation sequencer (e.g., Ion S5/XL system, Life Technologies Japan Ltd.). RNA expression can be quantified on the basis of the number of reads (read count) prepared by the sequencing.

The probe or the primers for use in the measurement described above, which correspond to the primers for specifically recognizing and amplifying the target gene of the present invention or a nucleic acid derived therefrom, or the probe for specifically detecting the RNA or the nucleic acid derived therefrom, can be designed on the basis of a nucleotide sequence constituting the target gene. In this context, the phrase "specifically recognize" means that a detected product or an amplification product can be confirmed to be the gene or the nucleic acid derived therefrom in such a way that, for example, substantially only the target gene of the present invention or the nucleic acid derived therefrom can be detected in Northern blot, or, for example, substantially only the nucleic acid is amplified in RT-PCR.

Specifically, an oligonucleotide comprising a given number of nucleotides complementary to DNA consisting of a nucleotide sequence constituting the target gene of the present invention, or a complementary strand thereof can be used. In this context, the "complementary strand" refers to one strand of double-stranded DNA consisting of A:T (U for RNA) and/or G:C base pairs with respect to the other strand. The term "complementary" is not limited by the case of being a completely complementary sequence in a region with the given number of consecutive nucleotides, and can have preferably 80% or higher, more preferably 90% or higher, further more preferably 95% or higher identity of the nucleotide sequence. The identity of the nucleotide sequence can be determined by algorithm such as BLAST described above.

For use as a primer, the oligonucleotide can achieve specific annealing and strand extension. Examples thereof usually include oligonucleotides having a strand length of 10 or more bases, preferably 15 or more bases, more preferably 20 or more bases, and 100 or less bases, preferably 50 or less bases, more preferably 35 or less bases. For use as a probe, the oligonucleotide can achieve specific hybridization. An oligonucleotide can be used which has at least a portion or the whole of the sequence of DNA (or a complementary strand thereof) consisting of a nucleotide sequence constituting the target gene of the present invention, and has a strand length of, for example, 10 or more bases, preferably 15 or more bases, and, for example, 100 or less bases, preferably 50 or less bases, more preferably 25 or less bases.

In this context, the "oligonucleotide" may be DNA or RNA and may be synthetic or natural. The probe for use in hybridization is usually labeled for use.

In the case of measuring a translation product (protein) of target TARC gene of the present invention, a molecule which interacts with the protein, a molecule which interacts with the RNA, or a molecule which interacts with the DNA, a method such as protein chip analysis, immunoassay (e.g., ELISA), mass spectrometry (e.g., LC-MS/MS and MALDI-TOF/MS), one-hybrid method (PNAS 100, 12271-12276 (2003)), or two-hybrid method (Biol. Reprod. 58, 302-311 (1998)) can be used and can be appropriately selected depending on the measurement object.

For example, in the case of using the protein as a measurement object, the measurement is carried out by contacting an antibody against the expression product of the present invention with a biological sample, detecting a protein in the sample bound with the antibody, and measuring the level thereof. For example, according to Western blot, the antibody described above is used as a primary antibody, and an antibody which binds to the primary antibody and which is labeled with, for example, a radioisotope, a fluorescent material or an enzyme is used as a secondary antibody so that the primary antibody may be labeled, followed by the measurement of a signal derived from such a labeling material using a radiation meter, a fluorescence detector, or the like.

The antibody against the translation product may be a polyclonal antibody or a monoclonal antibody. These antibodies can be produced in accordance with a method known in the art. Specifically, the polyclonal antibody may be produced by using a protein which has been expressed in *E. coli* or the like and purified in accordance with a routine method, or synthesizing a partial polypeptide of the protein in accordance with a routine method, and immunizing a nonhuman animal such as a house rabbit therewith, followed by obtainment from the serum of the immunized animal in accordance with a routine method.

On the other hand, the monoclonal antibody can be obtained from hybridoma cells prepared by immunizing a nonhuman animal such as a mouse with a protein which has been expressed in *E. coli* or the like and purified in accordance with a routine method, or a partial polypeptide of the protein, and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

In this way, the expression level of the TARC gene or the expression product thereof in SSL collected from a test subject is measured, and childhood atopic dermatitis is detected on the basis of the expression level. The detection is specifically performed by comparing the measured expression level of the TARC gene of the present invention or the expression product thereof with a control level.

In the case of analyzing expression levels of a plurality of target genes by sequencing, as described above, read count values which are data on expression levels, RPM values which normalize the read count values for difference in the total number of reads among samples, values obtained by the conversion of the RPM values to logarithmic values to base 2 (Log₂RPM values) or logarithmic values to base 2 of RPM plus integer 1 (Log₂(RPM + 1) values), or normalized count values obtained using DESeq2 or logarithmic values to base 2 of count value plus integer 1 (Log₂(count + 1) values) are preferably used as an index. Also, values calculated by, for example, fragments per kilobase of exon per million reads mapped (FPKM), reads per kilobase of exon per million reads mapped (RPKM), or transcripts per million (TPM) which are general quantitative values of RNA-seq may be used. Signal values obtained by microarray method or corrected values thereof may be used. In the case of analyzing an expression level of only the target TARC gene by RT-PCR or the like, an analysis method of converting the expression level of the target gene to a relative expression level based on the expression level of a housekeeping gene (relative quantification), or an analysis method of quantifying an absolute copy number using a plasmid containing a region of the target gene (absolute quantification) is preferred. A copy number obtained by digital PCR may be used.

In this context, examples of the "control level" include an expression level of the TARC gene or the expression product thereof in a healthy child. The expression level of the healthy child may be a statistic (e.g., a mean) of the expression level of the TARC gene or the expression product thereof measured from a population of healthy children. Depending on the purpose of detection, the expression level of the TARC gene or the expression product thereof in a child with mild atopic dermatitis or a child with moderate atopic dermatitis may be used as the "control level".

The detection of childhood atopic dermatitis according to the present invention may be performed through an increase and/or decrease in the expression level of the TARC gene or the expression product thereof. In this case, the expression level of the TARC gene or the expression product thereof in SSL derived from a test subject is compared with a cutoff value (reference value) of the TARC gene or the expression product thereof.

The cutoff value can be determined by various statistical analysis approaches. Examples thereof include values based on ROC curve (receiver operatorating characteristic curve) analysis (e.g., Youden's index and distance values from the top left corner, the coordinate (0,1) in the ROC curve).

The ROC curve is prepared by plotting the probability of producing positive results in positive patients (TPF: true position fraction, sensitivity) on the ordinate against a value (FPF: false position fraction) of 1 minus the probability of producing negative results in negative patients (specificity) on the abscissa while changing a threshold to determine what value in examination results is regarded as being abnormal, i.e., a cutoff point, as a parameter.

A cutoff point to be adopted as the cutoff value in the prepared ROC curve can be determined depending on the severity of the disease, the positioning of examination, and other various conditions. Typically, if a point at which the false positive fraction is low is adopted as the cutoff point, the number of negative patients regarded as being positive is decreased whereas many positive patients are excluded. As a result, the sensitivity is reduced. On the contrary, if the sensitivity is enhanced, the false positive fraction of negative patients is elevated.

In general, in order to enhance both the sensitivity and the specificity (to bring them closer to 1), the cutoff value is set to a value that gives a point closest to (0,1) on the ROC curve, or a value at which ["true positive (sensitivity)" - "false positive (1 - specificity)"] is maximized (Youden index).

The test kit for detecting childhood atopic dermatitis according to the present invention contains a test reagent for measuring an expression level of the TARC gene or an expression product thereof in SSL separated from a patient. Specific examples thereof include a reagent for nucleic acid amplification and hybridization containing an oligonucleotide (e.g., a primer for PCR) which specifically binds (hybridizes) to the TARC gene or a nucleic acid derived therefrom, and a reagent for immunoassay containing an antibody which recognizes an expression product (protein) of the TARC gene. The oligonucleotide, the antibody, or the like contained in the kit can be obtained by a method known in the art as mentioned above.

The test kit can contain, in addition to the antibody or the nucleic acid, a labeling reagent, a buffer solution, a chromogenic substrate, a secondary antibody, a blocking agent, an instrument necessary for a test, a control reagent for use as a positive control or a negative control, a tool for collecting SSL (e.g., an oil blotting film for collecting SSL), a reagent for preserving the collected SSL, a container for preservation, a reagent for extracting and/or purifying RNA from the collected SSL, and the like.

Aspects and preferred embodiments of the present invention will be given below.
<1> A method for detecting childhood atopic dermatitis in a test subject, comprising a step of measuring an expression level of TARC gene or an expression product thereof in skin surface lipids collected from the test subject.
<2> The method according to <1>, wherein the expression level of the gene or the expression product thereof is measured as an expression level of mRNA.
<3> The method according to <1> or <2>, wherein the presence or absence of childhood atopic dermatitis or a degree of progression thereof is evaluated by comparing the measurement value of the expression level with a reference value of the gene or the expression product thereof.
<4> The method according to <3>, wherein the degree of progression of childhood atopic dermatitis is low grade or intermediate grade.
<5> The method according to <3> or <4>, wherein the degree of progression of childhood atopic dermatitis is a degree of progression (severity) based on an EASI score or an IGA score.
<6> The method according to any of <1> to <4>, wherein the test subject is a 0- to 5-year-old child.
<7> The method according to any of <1> to <5>, wherein the test subject is a child under the age of 2.
<8> Use of TARC gene or an expression product thereof derived from skin surface lipids collected from a test subject, as a marker for childhood atopic dermatitis.
<9> The use according to any of <8>, wherein the TARC gene or the expression product thereof is mRNA contained in skin surface lipids collected from the test subject.
<10> The use according to <8> or <9>, wherein the presence or absence of childhood atopic dermatitis or a degree of progression thereof is evaluated.
<11> The use according to <10>, wherein the degree of progression of childhood atopic dermatitis is low grade or intermediate grade.
<12> The use according to <10> or <11>, wherein the degree of progression of childhood atopic dermatitis is a degree of progression (severity) based on an EASI score or an IGA score.
<13> The use according to any of <8> to <12>, wherein the test subject is a 0- to 5-year-old child.
<14> The use according to any of <8> to <13>, wherein the test subject is a child under the age of 2.
<15> A test kit for detecting childhood atopic dermatitis, the kit being used in the method according to any of <1> to <7>, and comprising an oligonucleotide which specifically hybridizes to the gene or a nucleic acid derived therefrom, or an antibody which recognizes an expression product of the gene.

### Examples

Hereinafter, the present invention will be described in more detail with reference to Examples. However, the present invention is not limited by these examples.

### Example 1 Detection of TARC gene in RNA extracted from SSL

### 1) SSL collection

20 children with healthy skin (HL) (from 6 months after birth to 5 years old, male and female) and 16 children with atopic dermatitis (AD) (from 6 months after birth to 5 years old, male and female) were selected as test subjects. The atopic dermatitis children were each diagnosed as having low grade or intermediate grade atopic dermatitis in terms of the severity by a dermatologist. EASI scores (Hanifin et al., Exp dermatol. 10, 2001) were used in diagnosis. In accordance with the literature, children having a score of larger than 0 and smaller than 6 were regarded as being mild (low grade), and children having a score of 6 or larger and smaller than 23 were regarded as being moderate (intermediate grade) (Chopra et al., Br J Dermatol. 177, 2017). As a result, 9 subjects had low grade, and 7 subjects had intermediate grade. Among the test subjects, test subjects under the age of 2 were 14 HL subjects and 10 AD subjects (6 subjects with mild AD and 4 subjects with moderate AD).

Sebum was collected from the whole face (including an eruption site for AD) of each test subject using an oil blotting film (5 × 8 cm, made of polypropylene, 3M Company). Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted using QIAzol Lysis Reagent (Qiagen N.V.) in accordance with the attached protocol. On the basis of the extracted RNA, cDNA was synthesized through reverse transcription at 42°C for 90 minutes using SuperScript VILO cDNA Synthesis kit (Life Technologies Japan Ltd.). The primers used for reverse transcription reaction were random primers attached to the kit. A DNA library derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 3) Expression analysis of TARC gene

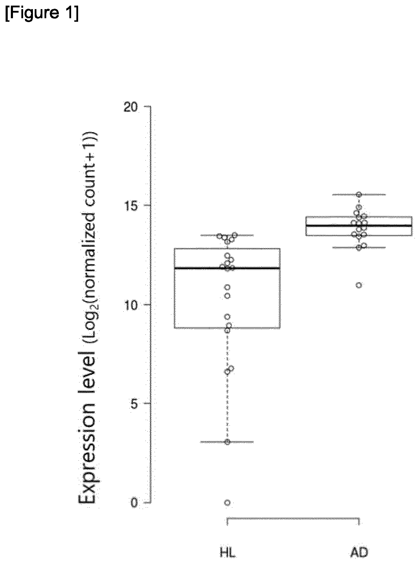

Data (read count values) on the expression level of RNA extracted from SSL of the healthy children, the children with mild atopic dermatitis, and the children with moderate atopic dermatitis measured in the above section 2) was obtained, followed by the normalization and differential expression analysis of the data using DESeq2. Genes which attained a corrected p value (FDR) of less than 0.05 in a likelihood ratio test in AD compared with the healthy subjects were selected. As a result, the expression of the TARC gene was significantly high in AD (FDR < 0.05), indicating that TARC in SSL, as in serum, is useful as a marker for detecting childhood atopic dermatitis. Difference in expression level based on logarithmic values to base 2 plus integer 1 of the normalized count values (Log₂(Normalized count + 1) values) is shown (Figure 1).

### 4) Verification of discrimination precision

ROC curves were prepared on the basis of the RNA expression level data (Log₂(Normalized count + 1) values) on TARC derived from SSL of the healthy children, the children with mild atopic dermatitis, and the children with moderate atopic dermatitis used in the above section 3). Cutoff values were set to values at which ["true positive (sensitivity)" - "false positive (1 - specificity)"] was maximized (Youden index). The cutoff values for distinguishing between healthy children and children with mild to moderate atopic dermatitis, between healthy children and children with mild atopic dermatitis, and between children with mild atopic dermatitis and children with moderate atopic dermatitis were set to 13.5, 12.5, and 13.8, respectively. As a result, as shown in Tables 1a to 1c below, precise determination was achieved with accuracy of 89%, 79%, and 87%, respectively, indicating that detection excellent in precision (sensitivity and specificity) is achieved as compared with conventional methods.

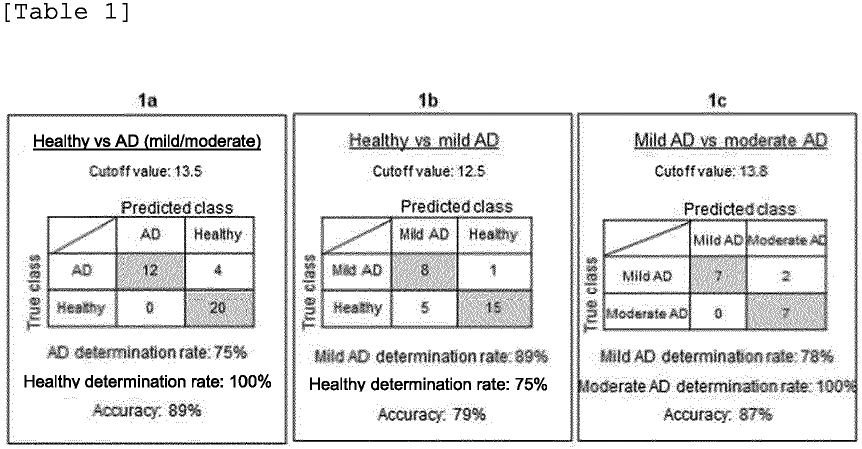

Results of performing the same verification as above targeting only children under the age of 2 are shown in Tables 2a to 2c. Cutoff values were set to values at which ["true positive (sensitivity)" - "false positive (1 - specificity)"] was maximized (Youden index). As a result, the cutoff values for distinguishing between healthy children and children with mild to moderate atopic dermatitis, between healthy children and children with mild atopic dermatitis, and between children with mild atopic dermatitis and children with moderate atopic dermatitis were set to numerical ranges from 12.5 to 12.8, from 12.5 to 12.8, and from 13.6 to 13.8, respectively. As shown in Tables 2a to 2c below, precise determination was thereby achieved with accuracy of 75%, 70%, and 90%, respectively, indicating that this approach is also useful for children under the age of 2 for which discrimination by conventional methods is difficult.

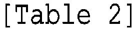

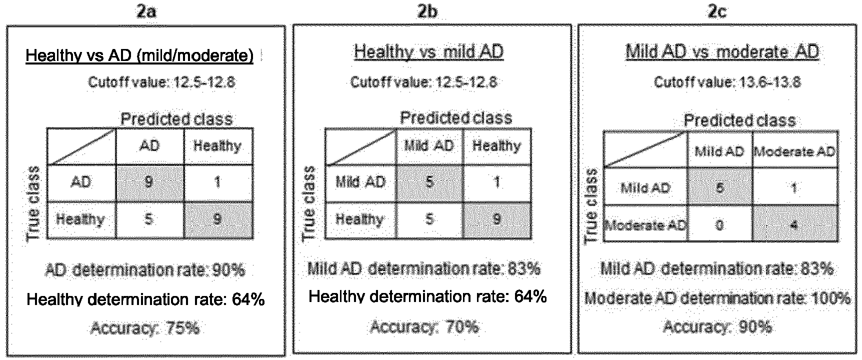

The following Reference Example will show that serum TARC levels and TARC expression levels from SSL-derived RNA do not always coordinate with each other by taking the case of adult atopic dermatitis patients as an example.

### Reference Example Detection of TRAC in adult-derived samples (SSL and serum)

### 1) SSL collection

14 healthy adult subjects (HL) (from 25 to 57 years old, male) and 29 adults having atopic skin (AD) (from 23 to 56 years old, male) were selected as test subjects. The test subjects with atopic dermatitis were each diagnosed as having mild or moderate atopic dermatitis in terms of severity by a dermatologist. Sebum was collected from the whole face of each test subject using an oil blotting film (5 × 8 cm, made of polypropylene, 3M Company). Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted using QIAzol Lysis Reagent (Qiagen N.V.) in accordance with the attached protocol. On the basis of the extracted RNA, cDNA was synthesized through reverse transcription at 42°C for 90 minutes using Superscript VILO cDNA Synthesis kit (Life Technologies Japan Ltd.). The primers used for reverse transcription reaction were random primers attached to the kit. A DNA library derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 3) Expression analysis of TARC gene

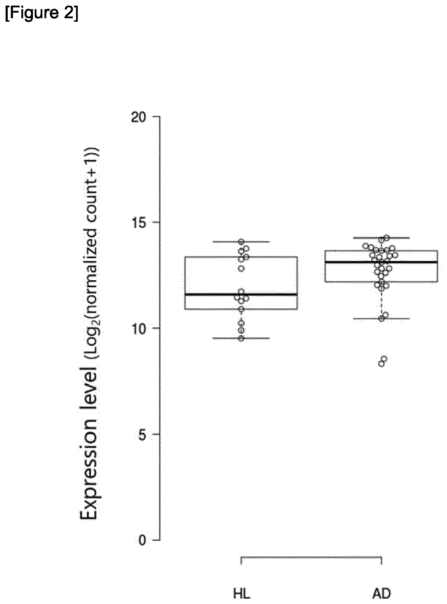

Data (read count values) on the expression level of RNA extracted from SSL of the healthy subjects and the AD patients measured in the above section 2) was obtained, followed by the normalization and differential expression analysis of the data using DESeq2. Genes which attained a corrected p value (FDR) of less than 0.05 in a likelihood ratio test in AD compared with the healthy subjects were selected. As a result, the TARC gene was not selected as the differentially expressed gene, and TARC in RNA derived from SSL of the adult AD patients exhibited no significant expression level based on the healthy subjects. Difference in expression level based on logarithmic values to base 2 of the normalized count values plus integer 1 (Log₂(Normalized count + 1) values) is shown (Figure 2).

### 4) Collection of blood

Blood was collected into a blood collection tube (Venoject(R) II-P, Terumo Corp.) by a physician or a nurse in accordance with a standard method from the test subjects (14 HL and 29 AD subjects) from which SSL was collected in the section 1). The blood collection tube was centrifuged (3,000 rpm, 15 min) at room temperature and refrigerated until analysis.

### 5) Analysis of serum TARC level

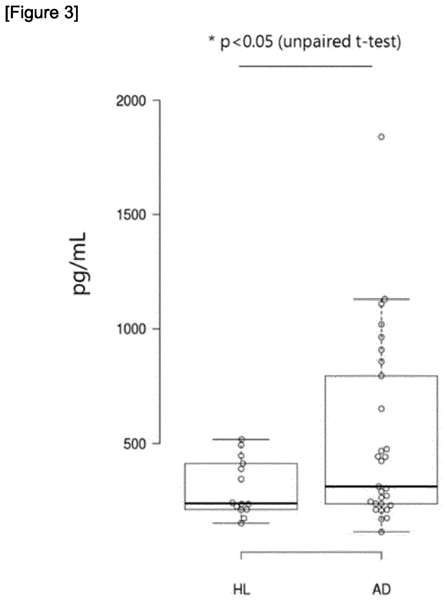

Serum TARC levels were analyzed by CLEIA (chemiluminescent enzyme immunoassay) cosigned to an external institution (LSI Medience Corp.). As a result of comparing the serum TARC protein levels of the healthy subjects and the AD patients, the serum TARC levels were significantly higher in the AD patients (unpaired t-test, p = 0.014) (Figure 3).

## Claims

1. A method for detecting childhood atopic dermatitis in a test subject, comprising a step of measuring an expression level of TARC gene or an expression product thereof in skin surface lipids collected from the test subject.

2. The method according to claim 1, wherein the expression level of the gene or the expression product thereof is measured as an expression level of mRNA.

3. The method according to claim 1 or 2, wherein the presence or absence of childhood atopic dermatitis or a degree of progression thereof is evaluated by comparing the measurement value of the expression level with a reference value of the gene or the expression product thereof.

4. The method according to claim 3, wherein the degree of progression of childhood atopic dermatitis is low grade or intermediate grade.

5. The method according to any one of claims 1 to 4, wherein the test subject is a 0- to 5-year-old child.

6. Use of TARC gene or an expression product thereof derived from skin surface lipids collected from a test subject as a marker for childhood atopic dermatitis.

7. The use according to claim 6, wherein the presence or absence of childhood atopic dermatitis or a degree of progression thereof is evaluated.

8. The use according to claim 6 or 7, wherein the test subject is a 0- to 5-year-old child.

9. A test kit for detecting childhood atopic dermatitis, the kit being used in a method according to any one of claims 1 to 5, and comprising an oligonucleotide which specifically hybridizes to the gene or a nucleic acid derived therefrom, or an antibody which recognizes an expression product of the gene.
